(19) Europäisches Patentamt
European Patent Office
Office européen des brevets

(11) **EP 1 500 926 B1**

(12) **EUROPÄISCHE PATENTSCHRIFT**

(45) Veröffentlichungstag und Bekanntmachung des Hinweises auf die Patenterteilung:
**19.08.2009 Patentblatt 2009/34**

(51) Int Cl.:
***G01N 27/416*** *(2006.01)*

(21) Anmeldenummer: **04016940.1**

(22) Anmeldetag: **19.07.2004**

(54) **Verfahren zur Kompensation der Alterung eines Sensors zur Erfassung einer Gaskonzentration**

Method for the compensation of aging of a sensor for measuring a gas concentration

Méthode pour la compensation du processus de vieillissement d'un capteur pour mesurer la concentration de gaz

(84) Benannte Vertragsstaaten:
**AT BE BG CH CY CZ DE DK EE ES FI FR GB GR HU IE IT LI LU MC NL PL PT RO SE SI SK TR**

(30) Priorität: **21.07.2003 AT 11342003**

(43) Veröffentlichungstag der Anmeldung:
**26.01.2005 Patentblatt 2005/04**

(73) Patentinhaber: **Vaillant GmbH**
**42859 Remscheid (DE)**

(72) Erfinder:
• **Altendorf, Frank**
**51061 Köln (DE)**
• **Ernst, Thomas**
**42857 Remscheid (DE)**
• **Fassbender, Hubert**
**41352 Korschenbroich (DE)**
• **Grüneberg, Richard**
**42283 Wuppertal (DE)**
• **Klepka, Michael**
**42369 Wuppertal (DE)**
• **Richter, Klaus**
**42857 Remscheid (DE)**

(74) Vertreter: **Hocker, Thomas**
**Vaillant GmbH**
**Berghauser Strasse 40**
**42859 Remscheid (DE)**

(56) Entgegenhaltungen:
**EP-A- 0 453 062          DE-A- 19 516 974**
**DE-A1- 4 344 961          DE-A1- 19 729 350**
**DE-C- 19 522 347**

**Beschreibung**

Die Erfindung bezieht sich auf ein Verfahren zur Kompensation der Alterung eines Sensors zur Erfassung einer Gaskonzentration. Derartige Sensoren werden beispielsweise in erdgas- oder erdölbetriebenen Heizungsanlagen eingesetzt, um ein optimales Brennstoff-Luft-Verhältnis einstellen zu können.

Gassensoren arbeiten üblicherweise bei Betriebstemperaturen oberhalb der Temperatur des zu untersuchenden Messgases. So werden beispielsweise Galiumoxid-Sensoren ($Ga_2O_3$) je nach Anwendung in einem Temperaturbereich zwischen 400 und 800°C eingesetzt. Dabei wird meist das Sensorelement durch ein auf seiner Rückseite angebrachtes elektrisches Heizelement beheizt.

Die Sensitivität eines Gassensors ist temperaturabhängig. Dies bedeutet, dass das Ausgangssignal des Sensors bei gleicher Gaszusammensetzung bei Veränderung der Sensortemperatur schwankt. Es ist daher notwendig, einen Gassensor bei einer konstanten Betriebstemperatur zu betreiben.

Durch Alterungsvorgänge im Sensorelement steigt - bei ansonsten gleichen Randbedingungen - der Widerstand R eines derartigen Gassensors.

Wird ein Sensor an einer Gleichstromquelle mit dem Konstantstrom I angeschlossen, so liegt gemäß der Formel

$$U = R * I$$

eine bestimmte Spannung U am Sensor an. Da dieses Signal sehr kleine Pegel aufweist, wird es verstärkt.

Steigt der Widerstand R über ein gewisses Maß an, so läge eine Spannung vor, die über der Versorgungsspannung liegt. Da dies nicht sein kann, liegt maximal die Versorgungsspannung an. Bei einer Veränderung der zu messenden Gaskonzentration ändert sich zwar der Widerstand des Sensors, jedoch liegt - aufgrund des begrenzten Spannungspotentials - keine entsprechende Änderung des Ausgangssignals, der Ausgangsspannung, vor.

Auch ist es möglich, dass einem bestimmten Bereich, der zu messender Größe ein bestimmter Bereich des Ausgangssignals zugeordnet wird. Verändert sich nun der Widerstand aufgrund von Alterungserscheinungen des Sensors, so liegt das Ausgangssignal außerhalb des Bereichs, der angezeigt oder verarbeitet werden kann.

Die DE 195 22 347 C1 beschreibt eine Regelung für einen Gassensor, welche die Betriebstemperatur des Sensors auch bei Drift des Heizwiderstandes stabilisiert.

Aus der DE 43 44 961 A1 ist eine beheizte Sauerstoffsonde im Abgasweg eines Verbrennungsmotors bekannt. An die Sonde wird eine Wechselspannung angelegt, um den Innenwiderstand zu messen. Die Temperatur der Sonde wird derartig verändert, bis der Sondeninnenwiderstand einem vorgegebenen Wert entspricht: Auch die DE 197 29 350 A1 befasst sich mit einer beheizten Sauerstoffsonde im Abgasweg eines Verbrennungsmotors. Im Gegensatz zu DE 43 44 961 A1 ist vorgesehen, dass die Temperatur der Sonde limitiert wird und stattdessen die Sollimpedanz des Sensors verändert wird.

Der Erfindung liegt daher die Aufgabe zugrunde, ein Verfahren zur Erkennung und Kompensation der Alterung eines Kohlenmonoxidsensors zur Erfassung einer Kohlenmonoxidkonzentration im Abgasweg einer Heizungsanlage zu schaffen.

Diese Aufgabe wird mittels eines Verfahrens mit den Merkmalen gemäß dem Anspruch 1 entweder dadurch gelöst, dass die Betriebstemperatur des Sensors erhöht wird. In bestimmten Fällen - es gibt auch Sensoren, bei denen der Widerstand mit steigender Temperatur zunimmt - kann es notwendig sein, dass die Betriebstemperatur reduziert wird, um denselben Effekt zu erzielen. Hierdurch verändert sich auch das Ausgangssignal, so dass das Signal wieder sinnvoll ausgewertet werden kann. Die Kompensation der Alterung wird dann durchgeführt, wenn die zu erfassende Gaskonzentration nicht vorliegt. Hierdurch liegen Referenzbedingungen vor. Die Erhöhung der Betriebstemperatur des Sensors wird beendet, sobald ein bestimmtes Messsignal unter- bzw. überschritten wird. Dieser Grenzwert muss nicht einem Extremwert des Ausgangssignals entsprechen. Vielmehr können auch gewisse Abstände zu den Grenzen eines Sollbereiches Berücksichtigung finden. Alternativ kann zur Kompensation der Alterung des Sensors auch das Übersetzungsverhältnis zwischen Messsignal und Ausgangssignal in einer Auswerteelektronik, einem Operationsverstärker oder dergleichen derart verändert werden, dass das Ausgangssignal wieder im Sollbereich liegt.

Gemäß den Merkmalen des abhängigen Anspruchs 2 kann nach einer bestimmten Zeit oder zu einem bestimmten Anlaß die Kompensation zumindest teilweise zurückgenommen werden, um festzustellen, ob die Veränderung nur temporär war und der Sensor sich wieder regeneriert hat. Ist dies der Fall, so wird die Temperatur wieder reduziert.

Gemäß den Merkmalen des abhängigen Anspruchs 3 wird - sobald ein entsprechender Grenzwert, der für vorangeschrittenen Verschleiß signifikant ist, überschritten ist - ein Signal zur Einleitung einer Wartung ausgegeben. Dies kann beispielsweise eine Anzeige an einem Display oder eine Mitteilung via Datenleitung oder Funkverbindung an einen Fachhandwerker sein.

Die Erfindung wird nun anhand der Zeichnungen detailliert erläutert. Hierbei zeigen

Fig. 1 eine sehr vereinfachte Schaltung mit dem Sensor,

Fig. 2 das Verhältnis zwischen einem Meßgas (hier Kohlenmonoxid CO) und dem Widerstand des Sensors bei konstanter Betriebstemperatur des Sensors,

Fig. 3 das Verhältnis zwischen der Betriebstemperatur des Sensors und dem Widerstand des Sensors bei konstantem Meßgas,

Fig. 4 das Verhältnis zwischen einem Meßgas (hier Kohlenmonoxid CO) und des Ausgangssignals des Sensors bei einer Konstantstromquelle und konstanter Betriebstemperatur des Sensors,

Fig. 5 das Verhältnis zwischen einem Meßgas (hier Kohlenmonoxid CO) und des Ausgangssignals des Sensors bei unterschiedlichen Betriebstemperaturen des Sensors entsprechend dem erfindungsgemäßen Verfahren und

Fig. 6 das Verhältnis zwischen Messgröße und Ausgangsgröße entsprechend dem zweiten unabhängigen Anspruch des erfindungsgemäßen Verfahrens.

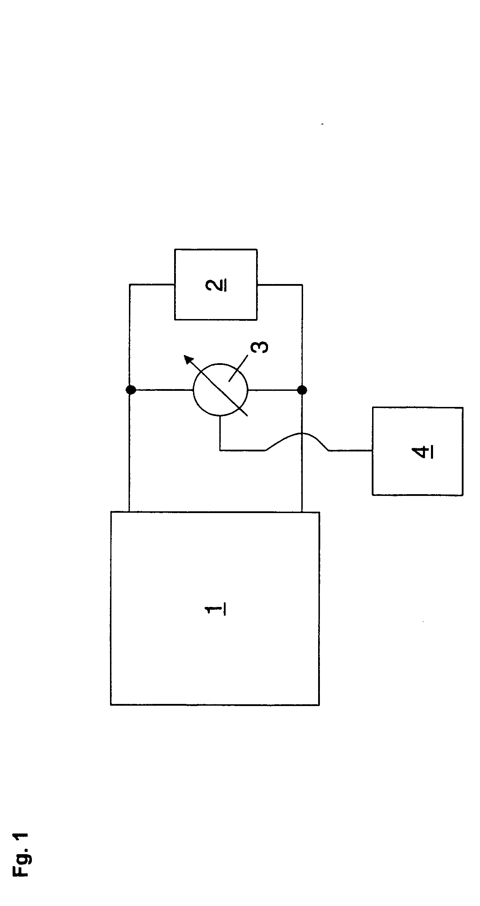

Figur 1 zeigt eine sehr vereinfachte Schaltung mit einem Sensor zur Erfassung der Gaskonzentration. Ein Konstantstromquelle 1 ist mit einem Sensor 2 zur Erfassung der Gaskonzentration vorzugsweise im Abgasweg einer Heizungsanlage, verbunden. Parallel zum Sensor 2 ist ein Spannungsmessgerät 3 angeordnet. Das Spannungsmessgerät 3 wiederum ist mit einem Operationsverstärker 4, der das Ausgangssignal verstärkt, verbunden.

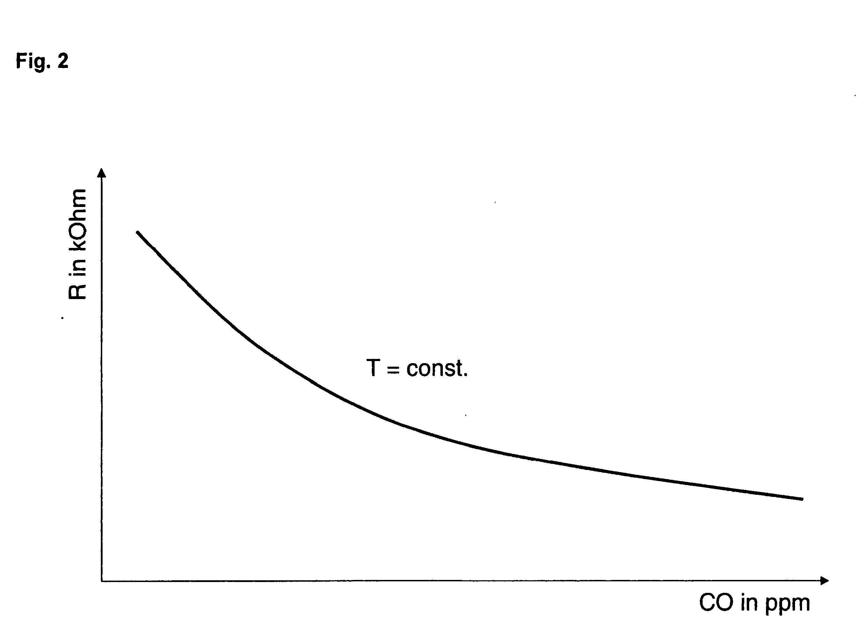

Figur 2 zeigt das Verhältnis zwischen einem Messgas, in diesem Fall Kohlenmonoxid (CO), und dem Widerstand eines Sensors zur Erfassung einer Gaskonzentration. Die Temperatur des Sensors sei hierbei konstant. Liegt kein Kohlenmonoxid vor, so hat der Widerstand einen bestimmten Wert. Mit dem Anstieg der Kohlenmonoxidkonzentration sinkt der Widerstand stetig.

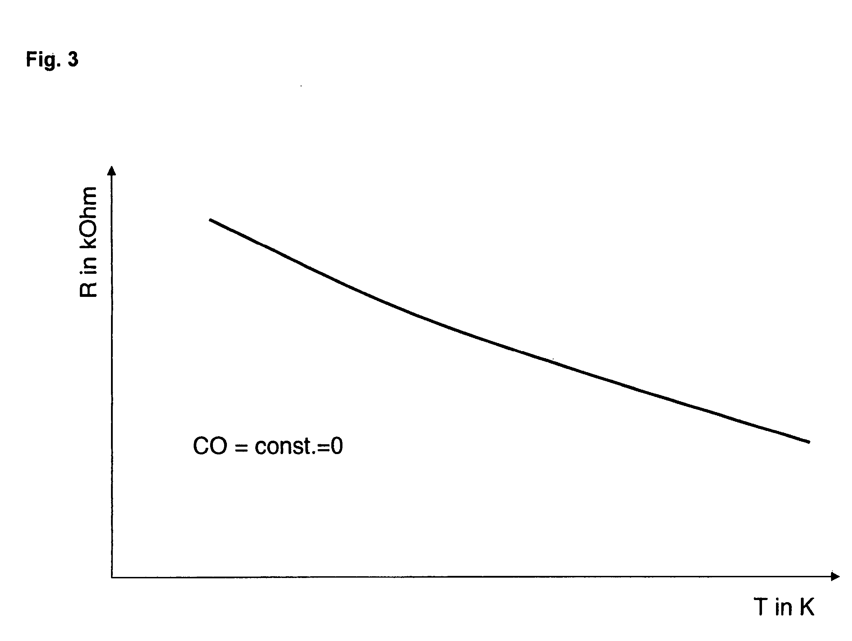

Figur 3 zeigt das Verhältnis zwischen der Betriebstemperatur des Sensors und dem Widerstand des Sensors bei konstantem Messgas. Man kann erkennen, dass mit steigender Temperatur der Widerstand sinkt.

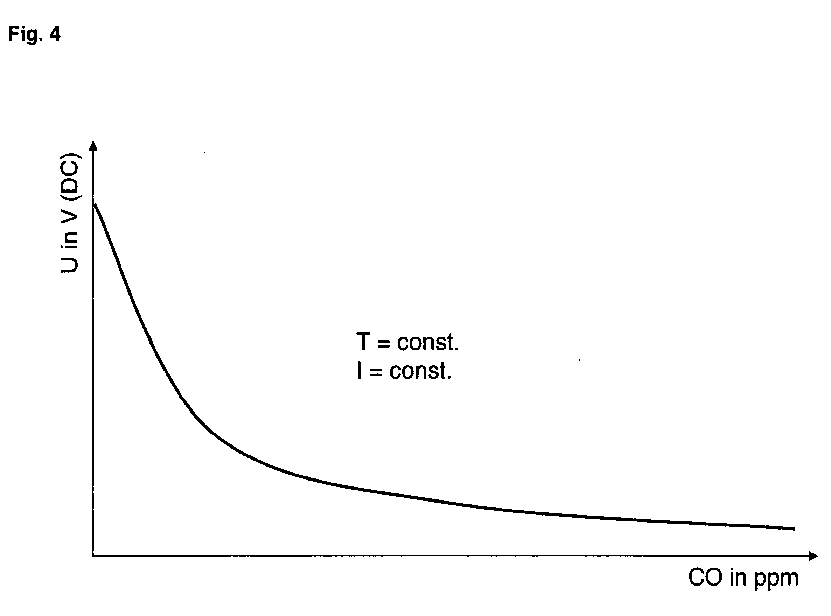

Figur 4 zeigt das Signal, dass an einem Spannungsmesser der parallel zu einem Gassensor angeschlossen ist, anliegt, sofern der Strom und die Temperatur konstant sind. Mit steigender Messgaskonzentration fällt die Spannung.

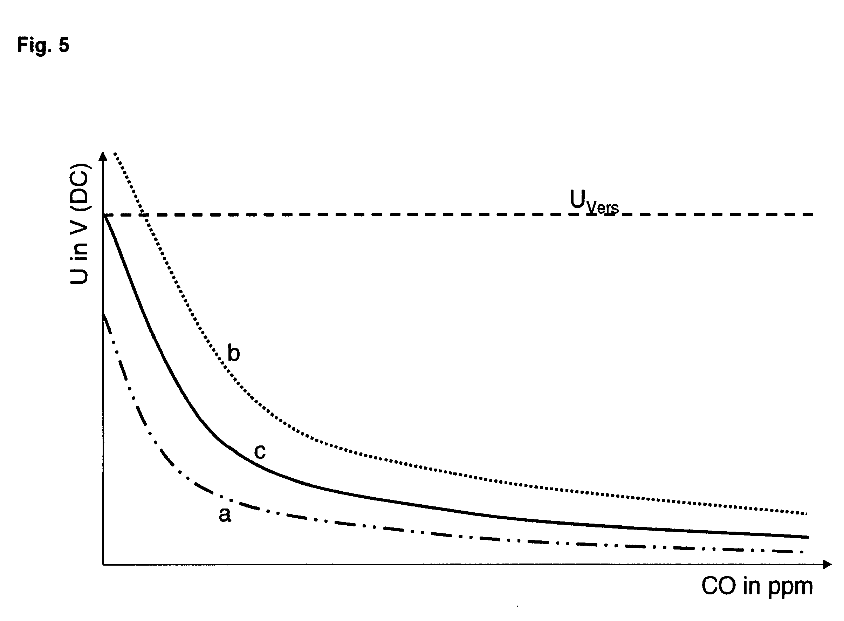

In Figur 5 ist dementsprechend der Spannungsverlauf gemäß des erfindungsgemäßen Verfahrens dargestellt. Linie a zeigt das Verhältnis zwischen Messgas und Ausgangsspannung im neuwertigen Zustand. Altert der Sensor und verändert sich demgemäß sein Widerstand, so würde eine Kennlinie gemäß b entstehen. Bei geringen Kohlenmonoxidkonzentrationen läge jedoch die Spannung höher als die Versorgungsspannung $U_{vers}$. Demgemäß wird die Betriebstemperatur des Sensors 2 erhöht, wodurch der Widerstand fällt. Demgemäß stellt sich ein Verhältnis gemäß der Linie c ein. Hierbei ist zu erkennen, dass sich ohne das Vorliegen des relevanten Gases (Kohlenmonoxid) eine Maximalspannung in Höhe der Versorgungsspannung einstellt.

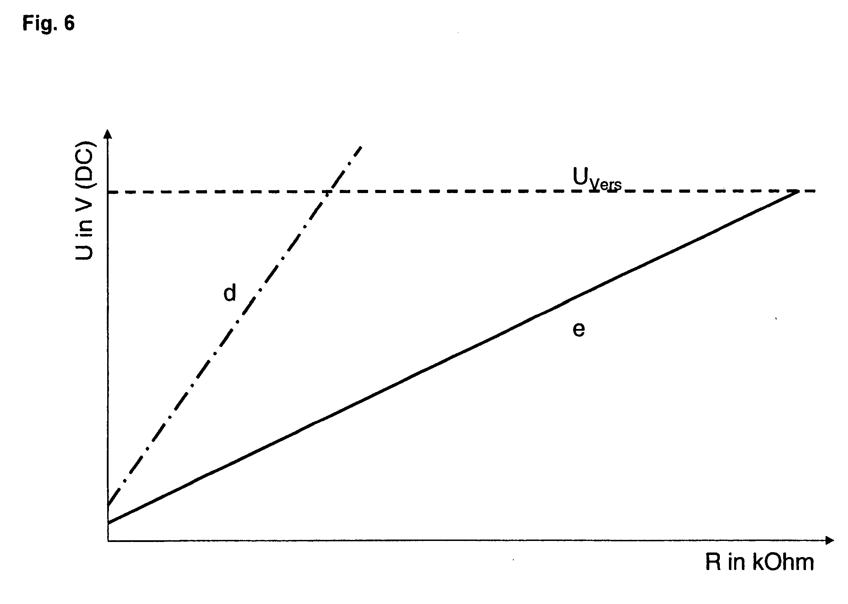

In Figur 6 ist das Verhältnis zwischen Messgröße und Ausgangsgröße entsprechend dem zweiten unabhängigen Anspruch des erfindungsgemäßen Verfahrens dargestellt. Die Linie d zeigt das Verhältnis im gealterten Zustand. Hieraus würde sich ergeben, dass die Ausgangsspannung höher als die Versorgungsspannung läge. Demgemäß wird das Verhältnis so angepasst, dass entsprechend Linie e auch bei hohem Messsignal das Ausgangssignal unterhalb der Versorgungsspannung liegt.

Um die Alterung des Sensors zu kompensieren und das System zu kalibrieren, wird an dem Sensor ein Gas vorbeigeführt, das keine oder eine definierte Konzentration des mittels des Sensors erfassten Gases enthält; z.B. reine Umgebungsluft wird an einem Kohlenmonoxidsensor vorbeigeführt. Es ist bekannt, dass - bezüglich der Kohlenmonoxidemissionen - der Widerstand des Sensors maximal ist (vgl. Fig. 2). Bei einer Konstantstromquelle ist demnach auch die gemessene Spannung maximal. Durch Veränderung der Sensortemperatur kann die gemessene Spannung verändert werden (vgl. Fig. 5). Die Sensortemperatur wird derartig verändert, dass eine definierte Ausgangsspannung bei oben genanntem Referenzgas vorliegt, vorzugsweise die Versorgungsspannung oder ein Wert geringfügig unterhalb dieser.

Der Vorteil des beschriebenen Verfahrens liegt darin, dass die Sensorstandzeit wesentlich verlängert werden kann. Zudem ist es möglich, eine hohe Auflösung auch bei gealtertem Sensor zu ermöglichen.

Zwar kann durch die Kompensation die Einsatzdauer eines Sensors verlängert werden, doch wird die Alterung zu einem späteren Zeitpunkt zum Ausfall führen. Daher ist optional vorgesehen, dass bei Überschreitung eines Grenzwertes die Regelung dies erkennt, und ein Signal zur Einleitung einer Wartung und Reparatur einleitet. Hierzu kann ein Telekommunikationsanschluss des Gerätes, in dem der Sensor sich befindet, dienen, über den z.B. ein mail oder eine SMS an einen Fachhandwerker versendet wird.

**Patentansprüche**

1. Verfahren zur Kompensation der Alterung eines beheizten Kohlenmonoxidsensors (2) zur Erfassung einer Kohlenmonoxidkonzentration im Abgasweg einer Heizungsanlage mit einem Operationsverstärker (4) zur Auswertung des Signals, **dadurch gekennzeichnet, dass** reine Umgebungsluft vorbeigeführt wird und bei Über- oder Unterschreitung eines vorgegebenen Widerstandswertes des Sensors (2) entweder die Betriebstemperatur des Sensors (2) erhöht oder reduziert wird bis beim Anschluss des Sensors (2) an eine Konstantstromquelle (1) eine definierte Ausgangsspannung oder beim Anschluss des Sensors (2) an eine Konstantspannungsquelle ein definierter Ausgangsstrom vorliegt oder das Übersetzungsverhältnis des Operationsverstärkers (4) zur Auswertung des Signals verändert wird, vorzugsweise dahingehend, dass das Ausgangssignal reduziert wird.

2. Verfahren zur Kompensation der Alterung eines beheizten Kohlenmonoxidsensors (2) gemäß Anspruch 1, **dadurch gekennzeichnet, dass** bei Erhöhung oder Reduktion der Betriebstemperatur des Sensors (2) nach einer bestimmten Betriebszeit des Sensors (2) die Betriebstemperatur des Sensors (2) wieder reduziert oder erhöht wird und in dem Fall, in eine Unterschreitung eines vorgegebenen Widerstandswertes des Sensors (2) vorliegt, der Sensor (2) wieder mit reduzierter oder erhöhter Betriebstemperatur betrieben wird.

3. Verfahren zur Kompensation der Alterung eines beheizten Kohlenmonoxidsensors (2) gemäß einem der Ansprüche 1 oder 2, **dadurch gekennzeichnet, dass** bei Überschreitung eines vorgegebenen Widerstandswertes des Sensors (2) eine Fehlermeldung auf einem Display und / oder über eine Datenleitung ein Wartungssignal ausgegeben wird.

**Claims**

1. A process for the compensation of aging of a heated carbon monoxide sensor (2) employed for sensing the carbon monoxide concentration in the exhaust path of a heating system using an operational amplifier (4) for evaluating the signal, **characterised in that** pure ambient air is passed by and, in the event that the resistance of the sensor (2) exceeds or falls short of a preset value, either the operating value of the sensor (2) is increased or reduced until a certain defined output voltage is present when the sensor (2) is connected to a constant current source (1), or a defined output current is present when the sensor (2) is connected to a defined output voltage, or that the transformation ratio of the operational amplifier (4) for signal evaluation is altered, preferably in such a manner that the output signal is reduced.

2. A process for the compensation of aging of a heated carbon monoxide sensor (2) in accordance with Claim 1, **characterised in that**, in the event of an increase in or reduction of the operating temperature of the sensor (2), after a certain operating time of the sensor (2) such operating temperature of the sensor (2) reduced again or increased again, and in the case that the resistance of the sensor (2) falls short of a preset value, that the sensor (2) is again operated at a reduced or increased operating temperature.

3. A process for the compensation of aging of a heated carbon monoxide sensor (2) in accordance with any of the Claims 1 or 2, **characterised in that**, in the event that the resistance of the sensor (2) exceeds a preset value, an error message is displayed on a display screen and/or a maintenance signal is output via a data transmission line.

**Revendications**

1. Procédé de compensation du vieillissement d'un capteur chauffé (2) de monoxyde de carbone, destiné à détecter une concentration de monoxyde de carbone dans le parcours des fumées d'une installation de chauffage, avec un amplificateur opérationnel (4) pour interpréter le signal, **caractérisé en ce qu'**on fait passer de l'air ambiant pur et, en cas de dépassement ou de sous-dépassement d'une valeur prescrite de résistance du capteur (2),
soit on augmente ou on réduit la température de fonctionnement du capteur (2) jusqu'à ce qu'on obtienne une tension de sortie définie, en cas de raccordement du capteur (2) à une source à courant constant (1), ou un courant de sortie défini, en cas de raccordement du capteur (2) à une source à tension constante,
soit on modifie le rapport d'amplification de l'amplificateur opérationnel (4) destiné à interpréter le signal, de préférence dans le sens d'une réduction du signal de sortie.

2. Procédé de compensation du vieillissement d'un capteur chauffé (2) de monoxyde de carbone, selon la revendication 1, **caractérisé en ce que**, en cas d'augmentation ou de réduction de la température de fonctionnement du capteur (2), on réduit ou on augmente à nouveau la température de fonctionnement du capteur (2) au bout d'une durée déterminée de fonctionnement du capteur (2), et en cas d'existence d'un sous-dépassement d'une valeur prescrite de résistance du capteur (2), le capteur (2) est à nouveau exploité avec une température de fonctionnement réduite ou augmentée.

3. Procédé de compensation du vieillissement d'un capteur chauffé (2) de monoxyde de carbone, selon la revendication 1 ou 2, **caractérisé en ce que**, en cas de dépassement d'une valeur prescrite de résistance du capteur (2), un message d'erreur est délivré sur un afficheur et/ou un signal de maintenance est délivré sur une ligne de transmission de données.

Fg. 1

Fig. 2

Fig. 3

CO = const.=0

R in kOhm

T in K

EP 1 500 926 B1

Fig. 4

EP 1 500 926 B1

Fig. 5

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19522347 C1
- DE 4344961 A1
- DE 19729350 A1